# FASCICULE DE BREVET EUROPEEN

(11) **EP 2 938 285 B1**
(45) Date de publication et mention de la délivrance du brevet: **24.08.2016**
(21) Numéro de dépôt: 13820840.0
(22) Date de dépôt: 20.12.2013
(51) Int. Cl.: A61B 50/20, A61B 50/30, A61B 17/86

(54) **ENSEMBLE COMPRENANT AU MOINS UN IMPLANT ET UN PREHENSEUR, ET PROCEDE DE PREPARATION A LA POSE DE L'IMPLANT D'UN ENSEMBLE DU TYPE PRECITE**
ANORDNUNG MIT MINDESTENS EINEM IMPLANTAT UND EINER GREIFVORRICHTUNG SOWIE VERFAHREN ZUR VORBEREITUNG DES EINSATZES DES IMPLANTATS AUS DIESER ANORDNUNG
ASSEMBLY INCLUDING AT LEAST ONE IMPLANT AND A GRIPPING DEVICE, AND METHOD OF PREPARATION FOR THE FIRRING OF HE IMPLANT OF AN ASSEMBLY OF THE AFOREMENTIONED TYPE

(30) Priorité: 31.12.2012 FR 1262987; 31.12.2012 FR 1262988
(43) Date de publication de la demande: 04.11.2015
(73) Titulaire: Neosteo, 44100 Nantes (FR)
(72) Inventeur: DEROUET, Guillaume, F-44420 La Turballe (FR); DECHELETTE, Maxime, F-44400 Rezé (FR)
(74) Mandataire: Godineau, Valérie
(86) Numéro de dépôt international: PCT/FR2013/053209
(87) Numéro de publication internationale: WO 2014/102490

(56) Documents cités:
- EP-A1- 2 392 286
- EP-B1- 1 241 998
- WO-A2-2009/024189
- DE-B- 1 074 819
- FR-A1- 2 959 216

## Description

La présente invention concerne un ensemble comprenant au moins un implant et un préhenseur, et un procédé de préparation à la pose de l'implant d'un ensemble du type précité.

Elle concerne plus particulièrement un ensemble du type comprenant :
- un implant présentant une extrémité distale d'introduction de l'implant dans le corps humain ou animal et une extrémité proximale, encore appelée tête de l'implant, apte de préférence à coopérer avec un outil de manoeuvre,
- un préhenseur dudit implant, ce préhenseur se présentant sous forme d'un corps creux, allongé, ouvert à au moins l'une, de préférence chacune de ses extrémités, et fendu longitudinalement sur au moins une partie, de préférence sur toute sa longueur, ce corps étant réalisé au moins partiellement en un matériau élastiquement déformable tendant à rappeler les bords de la fente dans le sens d'un rapprochement, cette fente dudit corps débouchant dans la cavité axiale dudit corps à l'intérieur de laquelle l'implant est apte à être inséré avec son extrémité distale faisant saillie de l'extrémité dite distale dudit corps.

De tels ensembles sont connus de l'état de la technique comme l'illustre, en particulier, le brevet EP 1.241.998, sur lequel est basée la forme en deux parties de la revendication 1. Des implants similaires sont également décrits dans la demande internationale WO 2009/024189 et la demande de brevet européen EP 2.392.286.

Les normes d'hygiène incitent à la mise en place de protocoles permettant de poser des implants sans qu'un contact tactile soit intervenu entre la main du chirurgien et l'implant.

Or les préhenseurs développés à ce jour posent notamment problème au moment du couplage de l'implant, tel qu'une vis, avec un outil de manoeuvre, tel qu'un tournevis, apte à permettre l'entraînement en rotation de l'implant pour son insertion dans une partie, telle qu'un os, du corps humain ou animal.

En effet, les préhenseurs, tels que celui décrit dans le brevet EP 1.241.998, sont réalisés en un matériau élastiquement déformable, tel que du caoutchouc, qui confère une souplesse au préhenseur. Cette souplesse facilite la déformation du préhenseur dans le sens d'un écartement des bords de la fente pour permettre la sortie de l'implant du préhenseur. Sans cette souplesse, une telle déformation est impossible.

Toutefois cette souplesse qui est un atout pour faciliter la séparation de l'implant du préhenseur devient un inconvénient lors du couplage de l'implant avec un outil de manoeuvre. En effet, cet outil de manoeuvre exerce un appui axial sur la tête de l'implant pour se coupler avec l'implant. Sous l'effet de cette poussée axiale, l'implant tend à glisser à l'intérieur du préhenseur de sorte qu'il existe un risque que l'implant s'échappe du préhenseur sans avoir été couplé à l'outil de manoeuvre. Il est alors nécessaire de manipuler directement l'implant, ce qui n'est pas recommandé. En outre, il existe un risque au moment où l'implant s'échappe du préhenseur qu'il atterrisse sur une surface non stérile.

Un but de la présente invention est donc de proposer un ensemble du type précité dont la conception permet de faciliter le couplage de l'implant avec un outil de manoeuvre sans toucher à l'implant.

A cet effet, l'invention a pour objet un ensemble comprenant au moins :
- un implant présentant une extrémité distale d'introduction de l'implant dans le corps humain ou animal et une extrémité proximale, encore appelée tête de l'implant, apte de préférence à coopérer avec un outil de manoeuvre,
- un préhenseur dudit implant, ce préhenseur se présentant sous forme d'un corps creux, allongé, ouvert à au moins l'une, de préférence chacune de ses extrémités, et fendu longitudinalement sur au moins une partie, de préférence sur toute sa longueur, ce corps étant réalisé au moins partiellement en un matériau élastiquement déformable tendant à rappeler les bords de la fente dans le sens d'un rapprochement, cette fente dudit corps débouchant dans la cavité axiale dudit corps à l'intérieur de laquelle l'implant est apte à être inséré avec son extrémité distale faisant saillie de l'extrémité dite distale dudit corps, caractérisé en ce que ledit ensemble comprend en outre des moyens d'aide à la séparation du préhenseur de l'implant sans toucher l'implant, ces moyens comprenant au moins un fourreau de réception de l'extrémité distale de l'implant, l'implant étant, à l'état introduit de son extrémité distale dans le fourreau, séparable de son préhenseur par déplacement relatif du fourreau et du préhenseur dans le sens d'une rupture de l'alignement entre fourreau et préhenseur jusqu'à passage de l'implant à travers la fente du préhenseur, ledit fourreau étant muni d'un siège d'appui de l'implant, à l'état introduit dudit implant dans le fourreau et à l'état séparé dudit implant de son préhenseur.

Grâce au fait que le fourreau forme, en coopération avec le préhenseur, une zone de saisie pour faciliter le dégagement de l'implant du préhenseur sans contact tactile avec l'implant, le préhenseur peut envelopper largement l'implant pendant les phases de stockage et de transport, ce que ne permet pas l'état de la technique qui doit laisser la tête de l'implant accessible.

Grâce au fait que le fourreau est muni d'un siège formant une assise de l'implant, il est possible, à l'aide d'un outil de manoeuvre, d'exercer une poussée axiale sur la tête de l'implant pour obtenir un couplage de l'implant et de l'outil par engagement de formes géométriques complémentaires sans provoquer un déplacement axial de l'implant au sein du fourreau.

Bien évidemment, le fourreau dit rigide est, au moins au niveau de son siège, réalisé en un matériau présentant une élasticité inférieure à celle du corps du préhenseur pour limiter la déformation du fourreau.

De préférence, ledit ensemble comprend en outre un élément de rangement de l'implant, préalablement à sa séparation du préhenseur, ledit élément de rangement comprenant au moins un emplacement de réception apte à loger d'une part, à emboitement au moins partiellement, au moins l'extrémité distale du corps du préhenseur, d'autre part, la partie distale de l'implant en saillie du préhenseur.

Grâce au fait que l'extrémité distale du corps de préhenseur est logée au moins partiellement à emboîtement dans un emplacement de réception de l'élément de rangement, l'effet de serrage par constriction du corps de préhenseur autour de l'implant peut être renforcé et les risques de sortie intempestive de l'ensemble préhenseur/implant d'un emplacement de réception au cours des phases de stockage ou de transport de l'ensemble sont réduits voire nuls.

De préférence, le fourreau est monté solidaire de l'élément de rangement.

Il en résulte une simplification de l'ensemble et une réduction du risque de perte du fourreau. En outre, lorsque l'ensemble comporte plusieurs préhenseurs associés chacun à un implant, un même fourreau peut servir à l'ensemble des couples préhenseur/implant.

De préférence, l'élément de rangement est formé au moins d'un plateau muni d'au moins deux cavités, au moins l'une des cavités correspondant à un emplacement de réception d'un préhenseur pré-équipé de son implant, tandis qu'au moins l'une des cavités forme au moins une partie dudit fourreau.

De préférence, le corps du préhenseur est un corps tubulaire épaulé, ledit épaulement, dit périphérique externe, depuis l'extrémité distale en direction de l'extrémité proximale dudit corps, formant une butée de limitation d'introduction du corps à l'intérieur d'un emplacement de réception de l'élément de rangement.

La partie du préhenseur qui s'étend entre l'extrémité proximale et l'épaulement du préhenseur forme donc la partie de saisie du préhenseur, à l'état rangé du préhenseur et de l'implant associé dans l'élément de rangement, tandis que la partie de préhenseur qui s'étend entre l'épaulement et l'extrémité distale du préhenseur est la partie du préhenseur logée au moins partiellement à emboîtement dans l'emplacement de réception de l'élément de rangement. Cette partie est donc non directement accessible. Comme elle jouxte la partie distale découverte de l'implant, elle-même logée dans l'emplacement de réception de l'élément de rangement, les possibilités d'un contact tactile de la main de l'opérateur avec l'implant au moment de la saisie du préhenseur en vue de son extraction de l'emplacement de réception de l'élément de rangement sont normalement nulles.

De préférence, la cavité axiale du corps du préhenseur comporte une zone de constriction de l'implant à l'intérieur de laquelle l'implant est apte à être retenu par serrage.

De préférence, la tête de l'implant est, à l'état inséré de l'implant dans la cavité du préhenseur, écartée de l'extrémité proximale du préhenseur.

A nouveau, cette disposition limite les risques de contact tactile entre la main du chirurgien et l'implant.

De préférence, les bords longitudinaux de la fente du préhenseur sont, à l'état non sollicité du préhenseur écartés d'une distance inférieure à 1 mm pour éviter tout contact à travers la fente de la main du chirurgien avec l'implant.

De préférence, l'ensemble comporte en outre des moyens d'identification de l'implant pris en sandwich entre une partie du préhenseur et l'élément de rangement à l'état inséré de l'implant et de son préhenseur associé dans un emplacement de réception de l'élément de rangement.

Cette disposition permet à un opérateur d'accéder aux moyens d'identification sans que ces derniers aient été préalablement manipulés par du personnel médical intervenant dans l'opération de pose de l'implant, tel que le chirurgien. L'invention a encore pour objet un procédé pour la préparation à la pose d'un implant d'un ensemble conforme à celui décrit ci-dessus, ledit implant et le préhenseur dudit ensemble étant préalablement associés,
caractérisé en ce que ledit procédé comprend au moins une étape de saisie manuelle du préhenseur associé à l'implant et d'introduction par son extrémité distale de l'implant dans ledit fourreau, une étape de séparation de l'implant de son préhenseur par déplacement relatif du fourreau et du préhenseur dans le sens d'une rupture de l'alignement entre fourreau et préhenseur jusqu'à passage de l'implant à travers la fente du préhenseur, une étape de retenue de l'implant dans le fourreau par appui sur le siège dudit fourreau jusqu'à saisie de l'implant par un outil de manoeuvre couplable, de préférence à force, avec la tête dudit implant.

Selon un mode de mise en oeuvre particulier du procédé dans lequel l'implant et le préhenseur associé sont préalablement stockés dans l'élément de rangement dudit ensemble, ledit procédé comprend, au cours de l'étape de saisie manuelle du préhenseur, une phase d'extraction du préhenseur et de l'implant associé de l'emplacement de réception de l'élément de rangement.

L'invention sera bien comprise à la lecture de la description suivante d'exemples de réalisation, en référence aux dessins annexés dans lesquels :
Les figures 1 à 6 représentent sous forme de vues en perspective les différentes étapes de manipulation d'un ensemble conforme à l'invention ;
La figure 7 représente une vue en coupe de l'élément de rangement prise au niveau d'une zone de l'élément de rangement comprenant trois cavités, deux des cavités correspondants chacune à un emplacement de rangement d'un préhenseur et de son implant, la troisième cavité correspondant à un fourreau de réception de l'implant ;
Les figures 8 à 10 représentent sous forme de vues en perspective les différentes étapes de manipulation d'une variante d'un ensemble conforme à l'invention ;
La figure 11 représente une vue en perspective du fourreau des figures 8 à 10 en position de stockage dans l'élément de rangement ;
La figure 12 représente une vue en coupe du fourreau de la figure 11 à l'état inséré d'un implant dans ledit fourreau.

Comme mentionné ci-dessus, l'ensemble, objet de l'invention, est destiné à permettre une manipulation de manière sûre d'un implant en vue de la pose dudit implant dans le corps humain ou animal en limitant les contacts tactiles entre implant et main du personnel médical d'intervention tout au cours de la manipulation.

Cet ensemble comprend un implant 1 réalisé ici sous forme d'une vis, telle qu'une vis d'ostéosynthèse. Cet implant 1 comprend une extrémité 11 distale d'introduction de l'implant dans le corps humain ou animal et une extrémité 12 proximale encore appelée tête de l'implant apte à coopérer avec un outil 9 de manoeuvre.

A cet effet, la tête de vis est munie d'une empreinte apte à être couplée par emboîtement de formes géométriques complémentaires avec l'extrémité de l'outil 9 de manoeuvre réalisé ici sous forme d'un tournevis.

L'ensemble comprend encore un préhenseur 2. Le préhenseur 2 se présente ici sous forme d'un corps 21 tubulaire de type manchon ou douille ouvert à chacune de ses extrémités et fendu longitudinalement sur toute sa longueur. Ce corps 21 est réalisé en élastomère.

Ce corps 21 est épaulé. Cet épaulement 5 est un épaulement périphérique externe depuis l'extrémité distale en direction de l'extrémité proximale dudit corps 21. Cet épaulement 5 forme une butée de limitation d'introduction du corps 21 à l'intérieur d'un emplacement 71 de réception d'un élément 7 de rangement qui sera décrit ci-après.

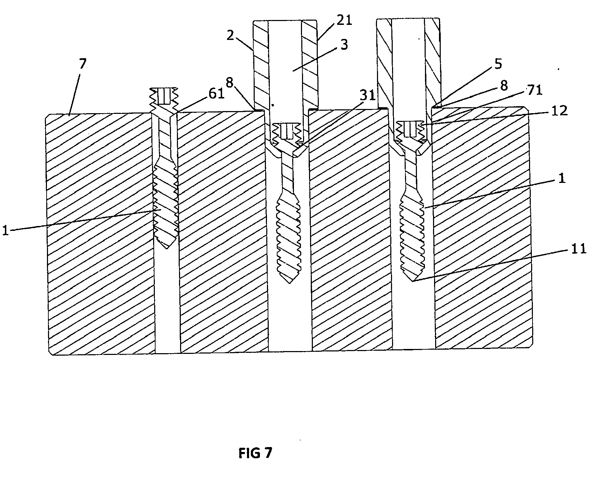

L'implant est disposé axialement à l'intérieur de la cavité 3 axiale du corps 21 de préhenseur. L'extrémité 11 distale de l'implant fait saillie de l'extrémité distale du corps 21 comme l'illustre la figure 7, et la tête 12 de l'implant est, à l'état inséré de l'implant 1 dans la cavité 3 du préhenseur 2, écartée de l'extrémité proximale du préhenseur 2.

La partie de saisie du préhenseur est donc formée, en particulier à l'état rangé du préhenseur dans un élément de rangement qui sera décrit ci-après, par la partie du préhenseur qui s'étend entre l'extrémité proximale et l'épaulement 5 du préhenseur 2.

La cavité 3 axiale du corps 21 du préhenseur comporte encore une zone 31 de constriction de l'implant 1 à l'intérieur de laquelle l'implant est apte à être retenu par serrage. Cette zone de constriction est ici formée par un étranglement de l'extrémité distale du corps de préhenseur. Cet étranglement s'étend, à l'état inséré de l'implant dans le préhenseur, immédiatement sous la tête de l'implant dans la zone de raccordement de la tête de l'implant au corps de l'implant comme l'illustre à nouveau la figure 7.

L'ensemble comprend encore des moyens d'aide à la séparation du préhenseur 2 de l'implant et un élément 7 de rangement de l'implant préalablement à sa séparation du préhenseur 2.

L'élément 7 de rangement comprend au moins un emplacement 71 de réception apte à loger, d'une part, à emboîtement au moins partiellement, au moins l'extrémité distale du corps 21 du préhenseur 2, d'autre part, la partie distale de l'implant 1 en saillie du préhenseur.

Dans les exemples représentés, l'élément 7 de rangement est formé d'un plateau épais qui peut constituer le corps d'une boîte fermée par un couvercle ou être inséré à l'intérieur d'une boîte d'instrumentation fermée par un couvercle. Ce couvercle, non représenté, vient à recouvrement de la face supérieure du plateau.

Cette face supérieure du plateau est munie d'une pluralité de cavités, en l'occurrence ici de douze cavités. Onze de ces cavités correspondent chacune à un emplacement 71 de réception d'un préhenseur 2 pré-équipé de son implant 1. A chaque fois, l'implant et le préhenseur sont disposés de sorte que la partie de saisie du préhenseur est disposée en saillie de la surface supérieure du plateau, tandis que la partie du préhenseur s'étendant entre épaulement et l'extrémité distale du préhenseur est logée à emboîtement dans la cavité.

La partie de l'implant en saillie du préhenseur qui correspond ici au corps de vis est également logée dans la cavité, de même que la tête de la vis qui est retenue dans le préhenseur par la zone de constriction du préhenseur.

La douzième cavité de l'élément de rangement forme ici tout ou partie des moyens d'aide à la séparation du préhenseur de l'implant. Ces moyens d'aide à la séparation du préhenseur comprennent, en effet, au moins un fourreau 6 de réception de l'extrémité 11 distale de l'implant.

Ce fourreau 6 comporte un siège 61 d'appui de l'implant 1 à l'état introduit dudit implant 1 dans le fourreau 6 et à l'état séparé dudit implant 1 de son préhenseur. La séparation s'opère comme suit :

A l'état introduit de l'extrémité distale de l'implant dans le fourreau 6, l'implant est séparé du préhenseur par déplacement relatif du fourreau 6 et de préhenseur 2 dans le sens d'une rupture de l'alignement entre fourreau 6 et préhenseur 2 jusqu'à passage de l'implant 1 à travers la fente 4 du préhenseur 2.

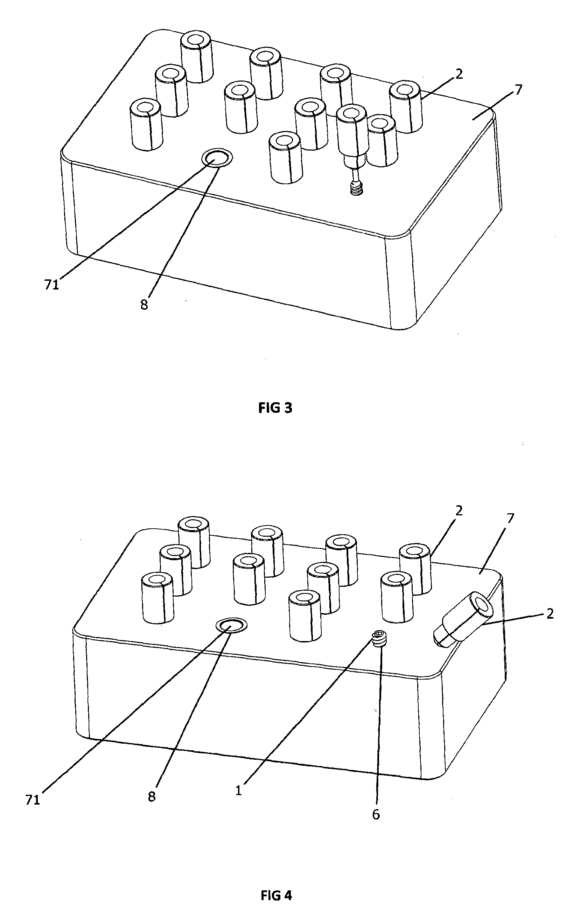

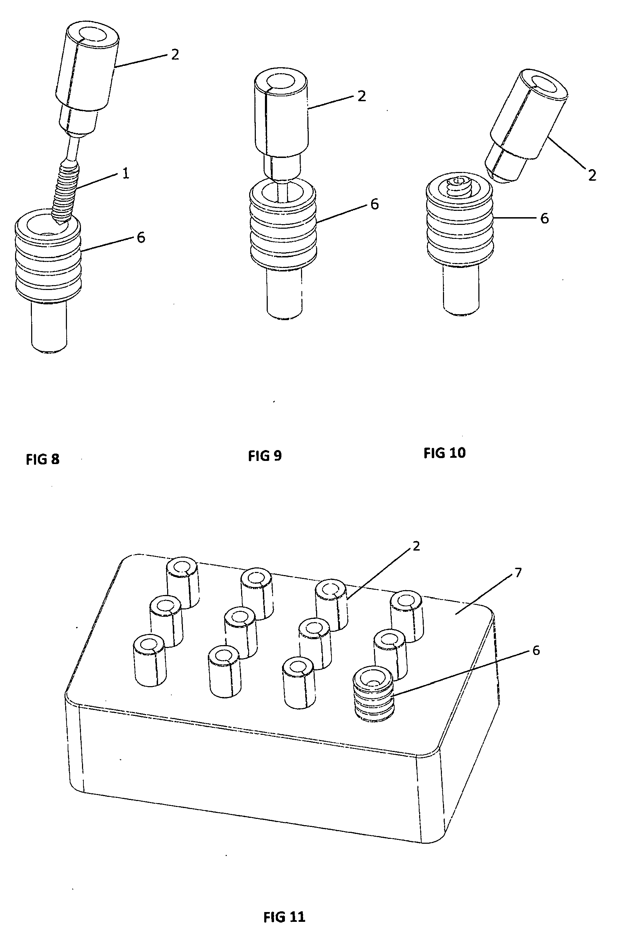

Ce déplacement relatif peut se traduire par un basculement ou pivotement du préhenseur par rapport au fourreau, en particulier par rapport à l'axe longitudinal du fourreau comme l'illustrent les figures 4 et 10. Ce basculement peut s'opérer par écartement de l'extrémité proximale du préhenseur de l'axe longitudinal du fourreau comme l'illustrent les figures, ou, par exemple, par écartement de l'extrémité distale du préhenseur de l'axe longitudinal du fourreau.

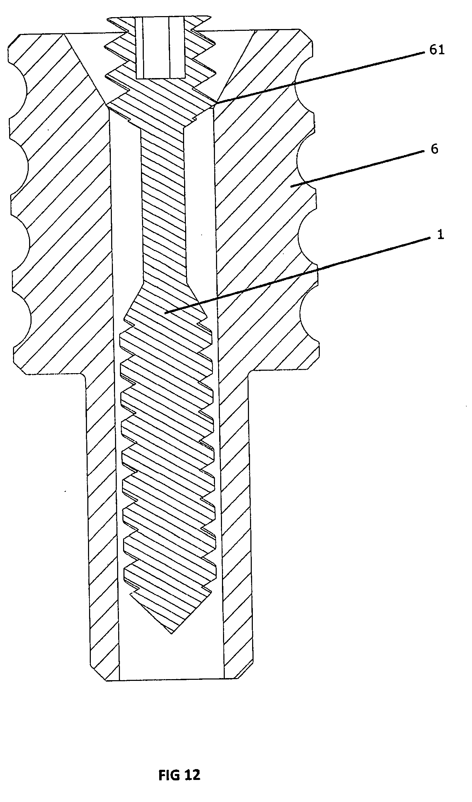

Une fois le préhenseur éliminé, l'implant reste dans le fourreau en appui sur le siège dudit fourreau comme l'illustrent, par exemple, les figures 7 et 12. Le siège formant une assise de l'implant, en particulier ici de la tête de l'implant, peut être formé par l'extrémité du fourreau par laquelle l'implant est introduit dans le fourreau (figure 7) ou par un étranglement ou un resserrement dans le passage délimité par ledit fourreau (figure 12).

Le siège ainsi réalisé permet à l'implant de prendre appui sur cette assise lorsque son extrémité proximale est soumise à une poussée axiale résultant de l'opération de couplage de l'implant avec l'outil 9 de manoeuvre. Généralement, le siège du fourreau est configuré pour retenir la tête de l'implant dans une position proche d'une extrémité ouverte du fourreau pour faciliter la coopération entre implant et outil de manoeuvre.

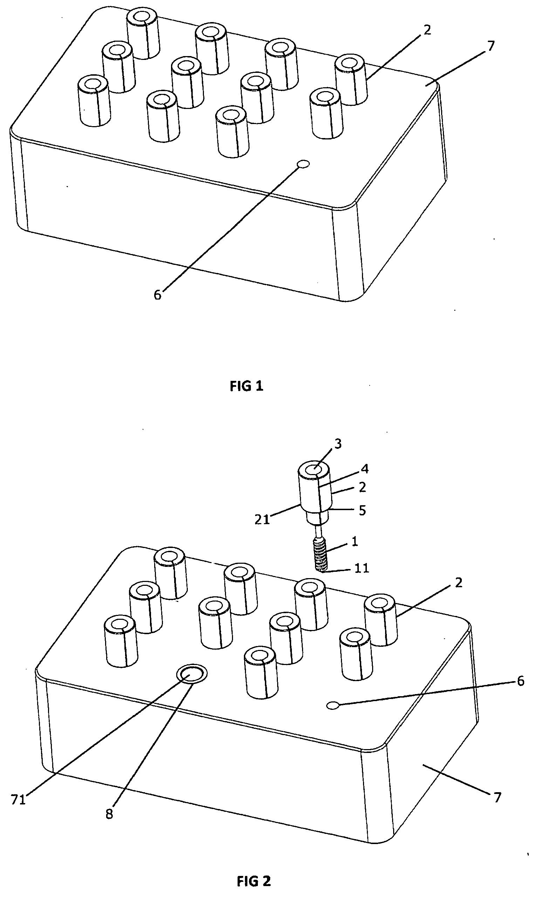

Il doit être noté que le fourreau peut indifféremment être un fourreau borgne ou traversant. De même, le fourreau peut être monté inamovible sur l'élément de rangement comme l'illustrent les figures 1 à 4 où le fourreau est formé par une simple cavité de l'élément de rangement.

Ce fourreau peut encore être formé par une pièce tubulaire insérable partiellement dans une cavité de l'élément de rangement comme l'illustrent les figures 10 et 11. Dans ce cas, le fourreau est couplable de manière amovible à l'élément de rangement. Au moins l'une des cavités de l'élément de rangement forme donc un espace de réception du fourreau.

Enfin, le fourreau peut, dans un mode de réalisation non représenté, être réalisé entièrement indépendant de l'élément de rangement.

L'ensemble peut encore comporter des moyens 8 d'identification de l'implant 1 pris en sandwich entre une partie du préhenseur 2 et l'élément 7 de rangement à l'état inséré de l'implant 1 et de son préhenseur 2 associé dans un emplacement 71 de réception de l'élément 7 de rangement.

Ces moyens 8 d'identification se présentent ici sous forme d'une rondelle positionnée autour d'un emplacement de réception de l'élément de rangement. Cette rondelle porte un marquage qui peut être lu une fois l'implant et son préhenseur désengagés de l'élément de rangement.

Au cours des phases de stockage et de transport, la rondelle est maintenue entre la face supérieure du plateau constitutif de l'élément de rangement et la surface épaulée du préhenseur.

Cette rondelle présente généralement une épaisseur de l'ordre 0,2 mm. Cette rondelle présente un diamètre intérieur supérieur au diamètre de la cavité de l'élément de rangement qu'elle entoure, et au diamètre de la partie de préhenseur qu'elle entoure, et de préférence inférieur au diamètre de la partie de saisie du préhenseur.

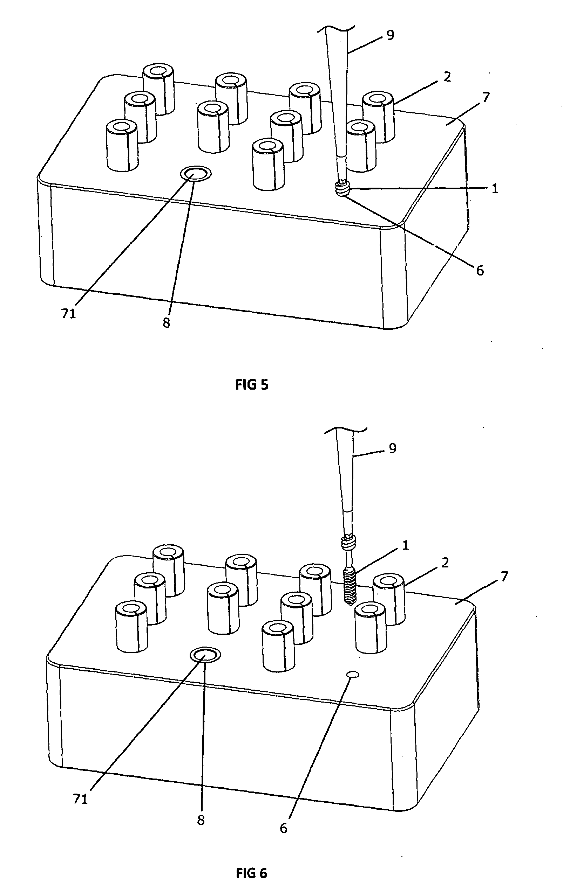

En pratique, la préparation et la pose d'un implant d'un ensemble tel que décrit ci-dessus s'opèrent comme suit :
L'implant étant stocké avec son préhenseur dans un élément de rangement, comme l'illustre la figure 1, l'opérateur, tel qu'un chirurgien, saisit dans un premier temps le préhenseur par sa partie de saisie pour extraire l'implant et le préhenseur de l'élément de rangement (figure 2), puis vient positionner l'extrémité distale de l'implant dans le fourreau (figure 3) avant de séparer le préhenseur de l'implant par basculement du préhenseur (figure 4). Le tournevis est inséré dans la tête de l'implant (figure 5) et la vis, manipulée à l'aide du tournevis couplé à la vis, est retirée du fourreau (figure 6). La vis est alors prête à être implantée dans un corps.

On note qu'à aucun moment l'opérateur n'a eu à toucher directement l'implant.

## Revendications

1. Ensemble comprenant au moins :
- un implant (1) présentant une extrémité (11) distale d'introduction de l'implant dans le corps humain ou animal et une extrémité (12) proximale, encore appelée tête (12) de l'implant, apte de préférence à coopérer avec un outil (9) de manoeuvre,
- un préhenseur (2) dudit implant. ce préhenseur (2) se présentant sous forme d'un corps (21) creux, allongé, ouvert à au moins l'une, de préférence chacune de ses extrémités, et fendu longitudinalement sur au moins une partie, de préférence sur toute sa longueur, ce corps (21) étant réalisé au moins partiellement en un matériau élastiquement déformable tendant à rappeler les bords de la fente (4) dans le sens d'un rapprochement, cette fente (4) dudit corps (21) débouchant dans la cavité (3) axiale dudit corps à l'intérieur de laquelle l'implant (1) est apte à être inséré avec son extrémité (11) distale faisant saillie de l'extrémité dite distale dudit corps (21),
**caractérisé en ce que** ledit ensemble comprend en outre des moyens d'aide à la séparation du préhenseur (2) de l'implant (1) sans toucher l'implant (1), ces moyens comprenant au moins un fourreau (6) de réception de l'extrémité (11) distale de l'implant (1), l'implant (1) étant, à l'état introduit de son extrémité (11) distale dans le fourreau (6), séparable de son préhenseur (2) par déplacement relatif du fourreau (6) et du préhenseur (2) dans le sens d'une rupture de l'alignement entre fourreau (6) et préhenseur (2) jusqu'à passage de l'implant (1) à travers la fente (4) du préhenseur (2), ledit fourreau (6) étant muni d'un siège (61) d'appui de l'implant (1), à l'état introduit dudit implant (1) dans le fourreau (6) et à l'état séparé dudit implant (1) de son préhenseur (2).

2. Ensemble selon la revendication 1,
**caractérisé en ce que** ledit ensemble comprend en outre un élément (7) de rangement de l'implant (1), préalablement à sa séparation du préhenseur (2), ledit élément (7) de rangement comprenant au moins un emplacement (71) de réception apte à loger, d'une part, à emboîtement au moins partiellement, au moins l'extrémité distale du corps (21) du préhenseur (2), d'autre part, la partie distale de l'implant (1) en saillie du préhenseur (2).

3. Ensemble selon la revendication 2,
**caractérisé en ce que** le fourreau (6) est monté solidaire de l'élément (7) de rangement.

4. Ensemble selon l'une des revendications 2 ou 3,
**caractérisé en ce que** l'élément (7) de rangement est formé au moins d'un plateau muni d'au moins deux cavités, au moins l'une des cavités correspondant à un emplacement (71) de réception d'un préhenseur (2) pré-équipé de son implant.

5. Ensemble selon la revendication 4,
**caractérisé en ce qu'**au moins l'une des cavités forme au moins une partie dudit fourreau (6).

6. Ensemble selon l'une des revendications 2 à 5,
**caractérisé en ce que** le corps (21) du préhenseur (2) est un corps tubulaire épaulé, ledit épaulement (5), dit périphérique externe, depuis l'extrémité distale en direction de l'extrémité proximale dudit corps (21), formant une butée de limitation d'introduction du corps (21) à l'intérieur d'un emplacement (71) de réception de l'élément (7) de rangement.

7. Ensemble selon l'une des revendications précédentes,
**caractérisé en ce que** la cavité (3) axiale du corps (21) du préhenseur (2) comporte une zone (31) de constriction de l'implant (1) à l'intérieur de laquelle l'implant est apte à être retenu par serrage.

8. Ensemble selon l'une des revendications précédentes,
**caractérisé en ce que** la tête (12) de l'implant (1) est, à l'état inséré de l'implant (1) dans la cavité (3) du préhenseur (2), écartée de l'extrémité proximale du préhenseur (2).

9. Ensemble selon l'une des revendications 1 à 8 prise en combinaison avec la revendication 2,
**caractérisé en ce qu'**il comporte en outre des moyens (8) d'identification de l'implant (1) pris en sandwich entre une partie du préhenseur (2) et l'élément (7) de rangement à l'état inséré de l'implant (1) et de son préhenseur (2) associé dans un emplacement (71) de réception de l'élément (7) de rangement.

10. Procédé pour la préparation à la pose d'un implant (1) d'un ensemble conforme à l'une des revendications 1 à 9, ledit implant (1) et le préhenseur (2) dudit ensemble étant préalablement associés,
**caractérisé en ce que** ledit procédé comprend au moins une étape de saisie manuelle du préhenseur (2) associé à l'implant (1) et d'introduction par son extrémité (11) distale de l'implant (1) dans ledit fourreau (6), une étape de séparation de l'implant (1) de son préhenseur (2) par déplacement relatif du fourreau (6) et du préhenseur (2) dans le sens d'une rupture de l'alignement entre fourreau (6) et préhenseur (2) jusqu'à passage de l'implant (1) à travers la fente (4) du préhenseur (2), une étape de retenue de l'implant (1) dans le fourreau (6) par appui sur le siège (61) dudit fourreau (6) jusqu'à saisie de l'implant (1) par un outil (9) de manoeuvre couplable, de préférence à force, avec la tête (12) dudit implant (1).

## Patentansprüche

1. Anordnung, umfassend mindestens:
- ein Implantat (1), das ein distales Einführende (11) des Implantats in den menschlichen oder tierischen Körper und ein proximales Ende (12), das auch als Kopf (12) des Implantats bezeichnet wird, das vorzugsweise imstande ist, im einem Manövrierwerkzeug (9) zusammenzuwirken, aufweist,
einen Greifer (2) des Implantats, wobei dieser Greifer (2) die Form eines länglichen hohlen, an mindestens einer Seite, vorzugsweise an jeder seiner Seiten, geöffneten Körpers (21) hat und längs über mindestens einen Teil, vorzugsweise über seine gesamte Länge, geschlitzt ist, wobei dieser Körper (21) mindestens teilweise aus einem elastisch verformbaren Material, das dazu tendiert, die Ränder des Schlitzes (4) in die Annäherungsrichtung zurückzuholen, hergestellt ist, wobei dieser Schlitz (4) des Körpers (21) in die axiale Kavität (3) des Körpers ausmündet, in deren Innern das Implantat (1) mit seinem distalen Ende (11), aus dem distalen Ende des Körpers (21) herausragend, einführbar ist,
**dadurch gekennzeichnet, dass** die Anordnung ferner Hilfsmittel für die Trennung des Greifers (2) vom Implantat (1) ohne das Implantat (1) zu berühren umfasst, wobei diese Mittel mindestens eine Empfangsmanschette (6) des distalen Endes (11) des Implantats (1) umfasst, wobei das Implantat (1) im eingeführten Zustand seines distalen Endes (11) in die Manschette (6) von seinem Greifer (2) durch relative Verlagerung der Manschette (6) und des Greifers (2) in Richtung einer Unterbrechung der Fluchtung zwischen Manschette (6) und Greifer (2) bis zum Durchgang des Implantats (1) durch den Schlitz (4) des Greifers (2) trennbar ist, wobei die Manschette (6) mit einem Stützsitz (61) des Implantats (1) im eingeführten Zustand des Implantat (1) in der Manschette (6) und im getrennten Zustand des Implantats (1) von seinem Greifer (2) ausgestattet ist.

2. Anordnung nach Anspruch 1,
**dadurch gekennzeichnet, dass** die Anordnung ferner ein Aufbewahrungselement (7) des Implantats (1) vor seiner Trennung vom Greifer (2) umfasst, wobei das Aufbewahrungselement (7) mindestens eine Empfangsstelle (71) umfasst, die imstande ist, zum einem durch mindestens teilweises Rasten mindestens das distale Ende des Körpers (21) des Greifers (2), zum anderen den aus dem Greifer (2) herausragenden distalen Teil des Implantats (1) aufzunehmen.

3. Anordnung nach Anspruch 2,
**dadurch gekennzeichnet, dass** die Manschette (6) mit dem Aufbewahrungselement (7) fest verbunden montiert ist.

4. Anordnung nach einem der Ansprüche 2 oder 3,
**dadurch gekennzeichnet, dass** das Aufbewahrungselement (7) von mindestens einer Platte gebildet ist, die mit mindestens zwei Vertiefungen ausgestattet ist, wobei mindestens eine der Vertiefungen einer Empfangsstelle (71) eines mit seinem Implantat vorausgestatteten Greifers (2) entspricht.

5. Anordnung nach Anspruch 4,
**dadurch gekennzeichnet, dass** mindestens eine der Vertiefungen mindestens einen Teil der Manschette (6) bildet.

6. Anordnung nach einem der Ansprüche 2 bis 5,
**dadurch gekennzeichnet, dass** der Körper (21) des Greifers (2) ein rohrförmiger Körper mit Absatz ist, wobei der äußere Umfangsabsatz (5) ab dem distalen Ende in Richtung des proximalen Endes des Körpers (21) einen Anschlag der Begrenzung des Einführens des Körpers (21) in das Innere einer Empfangsstelle (71) des Aufbewahrungselements (7) bildet.

7. Anordnung nach einem der vorangehenden Ansprüche,
**dadurch gekennzeichnet, dass** die axiale Vertiefung (3) des Körpers (21) des Greifers (2) eine Verengungszone (31) des Implantats (1) aufweist, in deren Innern das Implantat durch Spannen rückhaltbar ist.

8. Anordnung nach einem der vorangehenden Ansprüche,
**dadurch gekennzeichnet, dass** der Kopf (12) des Implantats (1) im eingesetzten Zustand des Implantats (1) in der Vertiefung (3) des Greifers (2) vom proximalen Ende des Greifers (2) beabstandet ist.

9. Anordnung nach einem der Ansprüche 1 bis 8, herangezogen in Kombination mit Anspruch 2,
**dadurch gekennzeichnet, dass** sie ferner Identifizierungsmittel (8) des Implantats (1) aufweist, die sich im eingesetzten Zustand des Implantats (1) und seines zugeordneten Greifers (2) in einer Empfangsstelle (71) des Aufbewahrungselements (7) sandwichmäßig zwischen einem Teil des Greifers (2) und des Aufbewahrungselements (7) befinden.

10. Verfahren für die Vorbereitung des Einführens eines Implantats (1) einer Anordnung nach einem der Ansprüche 1 bis 9, wobei das Implantat (1) und der Greifer (2) der Anordnung zuvor zugeordnet wurden,
**dadurch gekennzeichnet, dass** das Verfahren mindestens einen manuellen Schritt des Greifens des dem Implantat (1) zugeordneten Greifers (2) und des Einführens mit seinem distalen Ende (11) des Implantats (1) in die Manschette (6), einen Schritt des Trennens des Implantats (1) von seinem Greifer (2) durch relative Verlagerung der Manschette (6) und des Greifers (2) in Richtung einer Unterbrechung der Fluchtung zwischen Manschette (6) und Greifer (2) bis zum Durchgang des Implantats (1) durch den Schlitz (4) des Greifers (2), einen Schritt des Haltens des Implantats (1) in der Manschette (6) durch Abstützung auf dem Sitz (61) der Manschette (6) bis zum Greifen des Implantats (1) durch ein koppelbares Manövrierwerkzeug (9), vorzugsweise mit Kraft, mit dem Kopf (12) des Implantats (1) umfasst.

## Claims

1. An assembly comprising at least:
- an implant (1) having a distal end (11) for insertion of the implant into the human or animal body and a proximal end (12), also called head (12) of the implant, preferably able to cooperate with a maneuvering tool (9),
- a gripping device (2) for said implant, this gripping device (2) assuming the form of an elongate hollow body (21), open at least at one, preferably each of its ends, and with a longitudinal slit over at least part, preferably all of its length, this body (21) being at least partially made from an elastically deformable material tending to return the edges of the slit (4) in the direction coming closer together, this slit (4) of said body (21) emerging in the axial cavity (3) of said body inside which the implant (1) is able to be inserted with its distal end (11) protruding from said distal end of said body (21),
**characterized in that** said assembly further comprises means to assist with separating the gripping device (2) from the implant (1) without touching the implant (1), these means comprising at least one sheath (6) for receiving the distal end (11) of the implant (1), the implant (1) being, in the state with its distal end (11) inserted in the sheath (6), separable from its gripping device (2) by relative movement of the sheath (6) and the gripping device (2) in the direction of a break in the alignment between the sheath (6) and the gripping device (2) until the implant (1) passes through the slit (4) of the gripping device (2), said sheath (6) being provided with a bearing seat (61) of the implant (1), in the state of said implant (1) inserted in the sheath (6) and in the state of said implant (1) separated from its gripping device (2).

2. The assembly according to claim 1,
**characterized in that** said assembly further comprises a storage element (7) of the implant (1), prior to its separation from the gripping device (2), said storage element (7) comprising at least one receiving location (71) able to house, on the one hand, by at least partial nesting, at least the distal end of the body (21) of the gripping device (2), and on the other hand, the distal part of the implant (1) protruding from the gripping device (2).

3. The assembly according to claim 2,
**characterized in that** the sheath (6) is mounted secured to the storage element (7).

4. The assembly according to one of claims 2 or 3,
**characterized in that** the storage element (7) is formed by at least one plate provided with at least two cavities, at least one of the cavities corresponding to a location (71) for receiving a gripping device (2) pre-equipped with its implant.

5. The assembly according to claim 4,
**characterized in that** at least one of the cavities forms at least part of said sheath (6).

6. The assembly according to one of claims 2 to 5,
**characterized in that** the body (21) of the gripping device (2) is a shouldered tubular body, said shoulder (5), called outer periphery, from the distal end toward the proximal end of said body (21), forming a stop limiting the insertion of the body (21) inside a receiving location (71) for the storage element (7).

7. The assembly according to one of the preceding claims,
**characterized in that** the axial cavity (3) of the body (21) of the gripping device (2) includes a restriction zone (31) of the implant (1) inside which the implant is able to be retained by gripping.

8. The assembly according to one of the preceding claims,
**characterized in that** the head (12) of the implant (1) is, in the state of the implant (1) inserted in the cavity (3) of the gripping device (2), separated from the proximal end of the gripping device (2).

9. The assembly according to one of claims 1 to 8 combined with claim 2,
**characterized in that** it further includes means (8) for identifying the implant (1) sandwiched between a part of the gripping device (2) and the storage element (7) in the state of the implant (1) and its associated gripping device (2) inserted in a receiving location (71) of the storage element (7).

10. A method for preparing the placement of an implant (1) of an assembly according to one of claims 1 to 9, said implant (1) and the gripping device (2) of said assembly being associated beforehand,
**characterized in that** said method comprises at least one step for manual grasping of the gripping device (2) associated with the implant (1) and insertion, by its distal end (11), of the implant (1) in said sheath (6), a step for separating the implant (1) from its gripping device (2) by relative movement of the sheath (6) and the gripping device (2) in the direction of a break in the alignment between the sheath (6) and the gripping device (2) until the implant (1) crosses through the slit (4) of the gripping device (2), a step for retaining the implant (1) in the sheath (6) by bearing on the seat (61) of said sheath (6) until the implant (1) is grasped by a maneuvering tool (9) able to be coupled, preferably forcibly, with the head (12) of said implant (1).
